# EUROPEAN PATENT SPECIFICATION

(11) **EP 0 717 778 B1**
(45) Date of publication and mention of the grant of the patent: **17.10.2007**
(21) Application number: 93917602.0
(22) Date of filing: 19.07.1993
(51) Int. Cl.: C12N 15/57, C12N 9/54, C11D 3/386

(54) **HIGH ALKALINE SERINE PROTEASES**
HOCHALKALISCHE SERINPROTEASEN
SERINE-PROTEASES HAUTEMENT ALCALINES

(43) Date of publication of application: 26.06.1996
(73) Proprietor: GENENCOR INTERNATIONAL, INC., Palo Alto, California 94304 (US)
(72) Inventor: MULLENERS, Leonardus, Johannes, Sofie, Marie, NL-5121 SV Rijen (NL); MISSET, Onno, NL-2623 CK Delft (NL); VAN DER LAAN, Jan, Metske, NL-4839 AP Breda (NL); VAN GASTEL, Franciscus, Josephus, Cornelius, NL-3124 BG Schiedam (NL); BROEKHUIZEN, Cornelis, Petrus, NL-2281 VK Rijswijk (NL); BAAS, Erik, Jan, NL-1013 AG Amsterdam (NL)
(74) Representative: Brasnett, Adrian Hugh
(86) International application number: PCT/EP1993/001917
(87) International publication number: WO 1994/002618

(56) References cited:
- EP-A- 0 328 229
- EP-A- 0 405 901
- WO-A-89/06279

## Description

### INTRODUCTION

### Technical Field

The present invention relates to new high alkaline serine protease mutants having improved properties for use in detergents. These properties include improved stain removing ability in laundry detergent washing compositions, improved stain removing ability at low laundering temperature, improved stability in laundry detergents upon storage and improved stability in suds prepared from the detergents.

### Background of the invention

Use of enzymatic additives, in particular proteolytic enzymes, in detergent compositions to enable removal of protein based soilings has been amply documented. See for example the published European Patent Applications EP-A-0220921 and EP-A-0232269, U.S. Patents Nos. 4,480,037 and Re 30,602, and the article "Production of Microbial Enzymes", Microbial Technology, vol. 1 (1979) 281-311, Academic Press.

Detergent compositions, which are applied for hard surface cleaning, toilet cleaning, dish washing and laundry cleaning, may be in a powder, liquid or paste form. Laundry detergents are generally divided into two major types, liquids and powders.

Proteolytic enzymes are generally difficult to combine with detergent compositions. They must be stable and active during application, for example in removing proteinaceous stains from textile during washing at temperatures ranging from about 10°C to over 60°C. Furthermore they must be stable for prolonged periods of time during storage in the detergent product. Consequently, enzymes have to be stable and functional in the presence of sequestering agents, surfactants, high alkalinity, often bleaching agents, and elevated temperature. As there exist neither universal laundry detergents nor universal washing conditions (pH, temperature, sud-concentration, water hardness) that are used all over the world, the demands on enzymes may vary based on the type of detergent in which they are used and on the washing conditions.

A commercially important group of proteases is that of the so-called high alkaline proteases, derived from alkalophilic Bacilli. The commercially available high alkaline protease product MAXACAL^{®} (Gist-brocades/IBIS) contains the serine protease "PB92", derived from Bacillus novo sp. PB92 (see U.S. Patent Re. No. 30,602). Its amino acid sequence is disclosed in EP-A-0283075 and EP-A-0284126. Also SAVINASE^{®} (Novo-Nordisk) is a member of this group. SAVINASE contains the "Subtilisin 309" enzyme, which is derived from Bacillus strain NCIB 10147 (U.S. Patent No. 3,723,750). Its amino acid sequence is disclosed in WO 89/06279, where the strain is referred to as Bacillus lentus. The amino acid sequences of these two proteases appear to differ only at position 85 (taking the residue numbering of the PB92 protease, which corresponds to position 87 in the BPN' numbering), where PB92 has an asparagine ("N") in the one letter amino acid code) and "Subtilisin 309" a serine ("S").

Since the PB92 protease is active in stain removing at alkaline pH-values, it is commonly used as a detergent additive, together with detergent ingredients such as surfactants, builders and oxidizing agents. The latter agents are mostly used in powder form. The detergent additive may also contain other enzymes, for example amylases, cellulases and/or lipases, as far as they are compatible with the protease. PB92 protease has a high stain removing efficiency as compared to other proteases, such as the "classic" subtilisins which are well known in the art. This means that less PB92 protease is needed to obtain the same wash performance. Sensitivity to oxidation is an important drawback of the PB92 protease and all other known serine proteases used for application in detergents.

Originally the commercially available alkaline proteases such as MAXACAL^{®} were developed for application in detergents at enhanced temperatures in the range 40-60°C. However nowadays, because the growing emphasis on ecomomy, there is an ongoing tendency to switch to lower temperatures. As a consequence the lower wash performance at reduced temperatures, e.g. 15-25°C, is an important handicap of the excisting commercially alkaline proteases.

There are several ways of obtaining new enzymes for an intended application, which are all known to the skilled artisan. Modification of existing enzymes by protein engineering is likely to be the most popular and effective method nowadays.

The most specific way of obtaining modified enzymes is by site-directed mutagenesis, enabling specific substitution of one or more amino acids by any other desired amino acid. EP-A-0130756 exemplifies the use of this technique for generating mutant protease genes which can be expressed to give modified proteolytic enzymes. A very effective method is the oligonucleotide mediated site-directed mutagenesis, which allows a number of different mutations to be introduced at a specific part of a DNA sequence by using a single synthetic oligonucleotide preparation.

For a comprehensive summary of the various detergent compositions and enzymes, their physical forms, the conditions which the enzymes have to meet for optimal functioning, the problems and limitations of the currently available enzymes for use in detergent enzyme compositions, preparation and screening of mutant proteases, etc., reference may be made to EP-A-0328229, which is incorporated herein by reference.

WO 89/06279 claims inter alia mutants of the "Subtilisin 309" protease, in which one or more residues at the following positions are substituted (taking the original BPN' residue numbering): 6, 9, 11-12, 19, 25, 36-38, 53-59, 67, 71, 89, 104, 111, 115, 120, 121-122, 124, 128, 131, 140, 153-166, 168, 169-170, 172, 175, 180, 182, 186, 187, 191, 194, 195, 199, 218, 219, 222, 226, 234-238, 241, 260-262, 265, 268, or 275. The number of examples in this reference describing mutants which have been actually made and tested is restricted to only eight, while no more than four positions are involved. These mutants are: S153A, G195D, G195E, N218S, [G195E M222A], [G195E M222C], M222A, and M222C.

EPA-A-0328229 discloses and claims inter alia mutant proteases which have at least 70% homology with the amino acid sequence of PB92 serine protease and differ by at least one amino acid residue at a selected site corresponding to 32, 33, 48-54, 58-62, 94-107, 116-118, 123-134, 150, 152-156, 158-161, 164, 166, 169, 175-186, 197, 198 and 203-216, 235, 243 and 259 in said PB92 serine protease, and having improved wash performance and/or improved stability relative to said PB92 serine protease. This reference is exemplified by 69 mutants, in which 17 positions are involved.

EP 0 405 901 also relates to enzymatic detergent compositions, and discloses enzymes, produced by mutating genes for a number of subtilisin proteases and expressing the mutated genes in a suitable host, which exhibit improved wash performance.

Despite the progress which seems to have been made in the past few years, there is a continuing interest in the development of new proteolytic enzymes with improved properties which make them more attractive for use in detergents. These properties may include, but are not limited to, better wash performance, improved stain removing ability at low laundering temperature, improved stability upon storage, or improved stability while they are used.

### SUMMARY OF THE INVENTION

In one aspect the present invention provides new PB92 or Subtilisin 309 mutant serine protease having specific mutations, resulting in considerably improved properties which make them very suitable for application in detergents, especially laundry detergents. These PB92 or Subtilisin 309 mutants include mutations at positions 60, 87, 97, 99, 102, 116, 117, 126, 127, 128, 130, 133, 134, 154, 156, 158, 159, 160, 164, 166, 169, 175, 180, 182, 193, 197, 198, 203, 211, 212, and 216.

In a preferred embodiment of the invention there are provided PB92 and Subtilisin 309 mutants having a mutation at position 102, preferably in combination with at least one further mutation. Of these, the PB92 mutants [S99G, V102N] and [V102N, N198G] are most preferred.

In another aspect the invention provides new enzymatic detergent compositions, comprising a proteolytic enzyme product which contains at least one of such new mutant proteolytic enzyme, whether or not in conjuction with other enzymes, for example amylases, cellulases and lipases.

These and other aspects of the invention will be further outlined in the detailed description hereinafter.

### DETAILED DESCRIPTION OF THE INVENTION

By the term "improved properties" as used in this specification in connection with "mutant proteases" we mean proteolytic enzymes with improved wash performance or improved stability with retained wash performance, relative to the corresponding wild-type protease.

The term "wash performance" of mutant proteases is defined in this specification as the contribution of a mutant protease to laundry cleaning additional to the effect of the detergent composition without enzyme under relevant washing conditions.

The term "relevant washing conditions" is used to indicate the conditions, particularly washing temperature, time, washing mechanics, sud concentration, type of detergent and water hardness, actually used in households in a detergent market segment.

The term "improved wash performance" is used to indicate that the wash performance of a mutant protease, on weight basis, is at least greater than 100% relative to the corresponding wild-type protease under relevant washing conditions.

The term "retained wash performance" is used to indicate that the wash performance of a mutant protease, on weight basis, is at least 80% relative to the corresponding wild-type protease under relevant washing conditions.

The term "improved stability" is used to indicate better stability of mutant proteolytic enzymes in laundry detergents during storage and/or their stability in the sud, which includes stability against oxidizing agents, sequestering agents, autolysis, surfactants and high alkalinity, relative to the corresponding wild-type enzyme.

EP-A-0328229 describes a method in which the preparation of mutant proteases is combined with an efficient selection procedure on the performance of these proteases. The test system is based on the removal of protease sensitive stains from test swatches in a launderometer or tergotometer, imitating relevant washing conditions. Suitable test swatches are, for example, the commercially available EMPA swatches. (Eidgenössische Material Prüfungs und Versuch Anstalt, St. Gallen, Switzerland) artificially soiled with proteinaceous stains. Relevant stains on swatches for testing proteases include blood, grass, chocolate, and other proteinaceous stains. The reference also discloses that in this test system other relevant factors, such as detergent composition, sud concentration, water hardness, washing mechanics, time, pH and temperature, are controlled in such a way that conditions typical for household application in a certain market segment can be imitated.

Wash performance of proteases is conveniently measured by their ability to remove certain representative stains under appropriate test conditions. This ability can be suitably determined by reflectance measurements on the test cloths, after washing with and without enzymes in a launderometer or tergotometer. The laboratory application test system according to the invention is representative for household application when used on proteases which are modified by DNA mutagenesis.

In order to practice the present invention essentially the same method can be used for the preparation, screening and selection of further mutant enzymes derived from wild-type enzymes which are produced by alkalophilic Bacilli. Preferred mutants are those encoded by a gene derived from a wild-type gene encoding the PB92 serine protease or the Subtilisin 309 serine protease and which show improved properties under the test conditions mentioned above. Also genes encoding closely related serine proteases, preferably having a homology greater than about 70%, more particularly greater than about 90%, are very suitable.

It will be clear that either oligonucleotide aided site directed mutagenesis or region directed random mutagenesis can be used or any other suitable method for efficiently generating mutations in the protease gene of choice.

In accordance with the invention, various mutants were obtained with unexpectedly improved properties, i.e. a considerably higher wash performance, improved stain removing ability at low laundering temperature, or considerably improved storage stability with a similar or even better wash performance. These improvements were surprising, since they were neither suggested by, nor could they be derived in any way from the teaching of EP-A-328229 or any other prior art, either alone or when taken together.

The present invention therefore provides a mutant protease for use in detergents which comprises:
having greater than 90% homology with either the amino acid sequence of PB92 serine protease having the amino acid sequence : 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
or the amino acid sequence of Subtilisin 309 serine protease having the amino acid sequence: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-S-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
 in which the amino acid residue at a selected site corresponding to position V102 in said PB92 serine protease or said Subtilisin 309 serine protease is changed to A, E, G, H, I, L, M, N, P, Q, S, T or Y
having improved wash performance and/or improved stability relative to said PB92 serine protease or said Subtilisin 309 serine protease said improved wash performance being determined in a washing system having the following features: IEC-zeolite detergent Formulation April 1988 5.6 g/l; sud volume per beaker 200 ml; temperature 30 °C; time 30 min; Na-perborate.4aq. 1.4 g/l; TAED 210 mg/l; 2 EMPA 221 10 x 10cm clean swatch; 15 stainless steel balls (phi. 6 mm); 2mM Ca²⁺; 0.7 mM Mg²+; 0 mM Na CO₃; and 2 EMPA 116 or 2 EMPA 117 or 2 CFT As-3 CACAO 5 x 5 cm swatches.

A preferred group of mutant protease according to the invention are those mutants of PB92 or Subtilisin 309 protease which differ by at least one of the following mutations: [S99G, V1021], [S99G,V102L], [S99G,V102N], [V102A], [V102A,M216S], [V102E], [V102G], [V102H], [V102I], [V102I,G116V,S126V,P127M], [V102I,G116V,S126V,P127M], [V102I,S130G], [V102L], [V102L, G116V,S126V,P127M], [V102L,S13DG], [V102L,M216F], [V102L, M216S], [V102M], [V102N], [V102N,R164Y], [V102N,N193G], [V102N, N197T,N198G], [V102N N196G, Y203W], [V102N, Y203M], [V102N, L211E], [V102N, M216X, where X is any amino acid except M], [V102N, M216S] [V102P], [V102P, M216S], [V102Q], [V102Q, M216S], [V102S], [V102S, M216S], [V102T] and [V102Y], having improved wash performance and/or improved stability relative to said PB92 serine protease or said subtilisin 309 serine protease said improved wash performance being determined in a washing system having the following features: IEC-zeolite detergent Formulation April 1988 5.6 g/l; sud volume per beaker 200 ml; temperature 30°C ; time 30 min; Na-perborate.4aq. 1.4 g/l; TAED 270 mg/l; 2 EMPA 221 10 x 10cm clean swatch; 15 stainless steel balls (phi. 6 mm); 2mM Ca²⁺; 0.7 mM Mg²⁺; 0 mM NaCO₃; and 2 EMPA 116 or 2 EMPA 117 or 2 CFT As-3 CACAO 5 x 5 cm swatches.

Preferably, the mutant proteases according to the present invention are in substantially pure form.

Certain new mutant proteases show a considerably improved resistance to oxidation, whereas their wash performance is also better and in many cases significantly better than the wash performance of the corresponding wild-type protease. These mutant enzymes have in common that the methionine ("M") at position 216 is substituted by another amino acid, preferably serine ("S") or glutamine ("Q"). Also substitution by phenylalanine ("F") or alanine ("A"), is suitable. Further substitutions include the positions 60, 99, 102, 116, 127, 128, 130, 154, 156, 158, 197, 198, 203, 211 and 212. Preferred enzymes are those M216S and M216Q mutants which are further substituted at position 102 or at one or more of the positions 116, 126, 127 and 128. Also M216S and M216Q mutants with substitutions at positions 197, 198 and 203 are of particular interest. Preferred mutants are [N60E, M216S], [S99G, M216S], [V102A, M216S], [V102L, M216S], [Y102N, M216S], [V102P, M216S], [V102Q, M216S], [V102S, M216S], [G116V, S126L, P127Q, S128A, M216S], [G116V, S126N, P127S, S128A, M216S], [G116V, S126R, P127Q, S128D, M216S], [P127E, S128T, M216S], [V197T, M216S], [N198G, M216S], [Y203W, M216S], [L211E, M216S], [G116V, S126N, P127S, S128A, M216Q], [S126M, P127A, 1S28G, M216Q], [V102L, M216F].

It should be noted that EP-A-0328229 describes improved oxidation stability with retained wash performance of certain M216S and M216Q mutants of PB92 and similar high alkaline serine proteases. However this reference does not teach or suggest that the "216" mutants of PB92 or Subtilisin 309 with the above-defined mutations would result even in a significantly improved wash performance.

In another aspect of the invention certain new mutant proteases which are generally not oxidation resistant, show a considerably improved wash performance. These mutant enzymes have substitution at position 102. Preferred mutants are those which have at least two modifications. These modifications include the positions: 102 combined with at least one additional mutation at a position selected from the group comprising positions 87, 97, 116, 117, 126, 127, 128, 130, 133, 134, 154, 156, 158, 159, 160, 264, 166, 169, 175, 180, 182, 193, 197, 198, 203, 211 or 212, preferably with at least one additional mutation at a position selected from the group comprising positions 130, 164, 197, 198, 203 or 211. Preferred mutants are [S99G,V102N], [S99G,V102L], [S99G, V102I], [V102I, S130G], [V102L,S130G], [V102N,R164Y], [V102N,N198G], [V102N,N197T,N198G], [V102N,N198G,Y203W], [V102N, Y203W], [V102N,L211E], [V102I, G116V,S126V,P127M], [V102L, G116V, S126V, P127M]. Particularly preferred mutants are [S99G, V102N] and [V102N, N198G].

Certain new mutant PB92 and Subtilisin 309 proteases exhibit unexpected activity on cacao stains, which was in no way predictable from the prior art. Such mutant proteases have one or more substitutions at positions 102, 115, 117, 126, 127, 128, 133, 154, 156, 158, 159, 160, 164, 197, 198, 203, 211 and 216. Preferred mutants are those which have at least two modifications out of these defined positions. These modifications include the positions : 102 combined with at least one additional mutation at a position selected from the group comprising positions 164 or 211; 127 combined with at least one additional mutation selected from the group comprising positions 203 or 211 ; 154 and 160 ; 158 and 159. In addition, these modifications include position M216S and M216Q combined with at least one additional mutation at positions 102 or 211. Preferred mutants are : [V102N,R164Y], [V102N, L211E], [V102N, N198G], [P127E, Y203W], [P127E,L211E], S154G,S160G], [S154D, S160G], [S158E,I159L], [M216S,V102Q], [M216S,L211E]. In addition preferred mutants are the PB92 M216S mutants with further substitutions V102Q and L211E.

Certain new mutant PB92 and Subtilisn 309 proteases exhibit improved stain removing ability at lower laundering temperatures, e.g. about 20°C. These mutants have usually one or more substitutions in the PB92 or Subtilisin 309 enzyme at position 99, 102, 116, 126, 127, 128, 130, 160, 197, 198 and 203. Preferred mutants are those which have at least two modifications out of these defined positions. These modifications include the positions : 99, combined with at least one additional mutation at positions 102 or 130, preferably with a mutation at position 130; 102 combined with at least one additional mutation selected from the group comprising positions 197, 198 or 203, preferably with at least one additional mutation at positions 99 or 198, most preferably with an additional mutation at position 99 or 198; 126 combined with at least one additional mutation at positions 116, 127, 128 or 160, preferably 126 combined with 127. Preferred mutants are [S99G,S130G], [S99G, V102N], [S99G,V102I], [V102N,N198G], [V102N,Y203W], [V102N,V197I,N198G], [S126V, P127M], [S126F,P127N], [G116V,S126V,P127M,S160D], [G116V,S126L, P127Q,S128A,S160D].

Useful mutants may also be made by combining any of the mutations or sets of mutations described in this specification. Besides, it is possible to combine useful mutations as disclosed herein with mutations at other sites, which may or may not cause a substantial change in the properties of the enzyme.

To illustrate the significance of the approach used in this invention for obtaining new proteases suited for application in laundry detergents, i.e. by using representative laundry application testing as primary selection criterion, the results of the wash performance tests of mutant PB92 proteases were compared with biochemical parameters as usually determined in protein biochemical and enzymological research. These results allow the conclusion that any relation between parameters determining affinity for defined substrates and kinetics of the proteolytic reaction and wash performance is absent.

Therefore, it is of course also possible to combine two or more mutants with different properties in one enzyme product or in the same washing process. Such combination may or may not have a synergistic effect.

The invention comprises also the use of one or more mutant proteolytic enzymes, as defined hereinbefore, in a detergent composition or in a washing process. Such detergent composition may also contain one or more other enzymes, for example an amylase, cellulase or lipase which should be compatible with the protease or proteases of choice. The selection of the best combination of enzymes usually depends on the requirements and needs of the customer, but generally does not require inventive skill.

Finally, it will be clear that by deletions or insertions of the amino acids in the protease polypeptide chain, either created artificially by mutagenesis or naturally occurring in proteases homologous to PB92 protease or Subtilisin 309, the numbering of the amino acids may change. However, it is to be understood that positions homologous to amino acid positions of PB92 protease or Subtilisin 309 will fall under the scope of the claims.

The mutant proteases according to the invention can be made in essentially the same way as described in EP-A-0328229. Also, the preparation of the genes which encode the desired mutant proteases, the cloning and expression of said genes, the choice of a suitable host, the fermentation conditions, recovery, purification, screening and selection of the enzymes, etc., are essentially the same as described in EP-A-0328229 and are well within the skill of an ordinary worker.

The following Examples are offered by way of illustration and not by way of limitation.

### EXPERIMENTAL SECTION.

Materials and Methods which includes construction of the mutants, production of the mutants, purification, high performance liquid chromatography (HPLC) using cation exchange resin and gel filtration column, polyacrylamide gel-electrophoresis, active-site titration and determination of the kinetic parameters are similar or identical to those described in EP-A-0328229, except when stated otherwise. The mutants which are marked in the examples with the extension ^{+DTT} were purified and stored in the presence of 2 mM dithiothreitol (DTT).

### EXAMPLE 1

The wash performance of various PB92 protease mutants was determined in a specially developed washing test which is described in detail in EP-A-0328229. In addition to the sodium-tripolyphosphate (STPP) containing powder detergent IEC-STPP in this example also a non-phosphate containing powder detergent (IEC-zeolite) was used. The typical features of both test systems which were applied to test the wash performance of the new protease mutants are summarized below:

| Washing system | IEC-STPP | IEC-zeolite |
|---|---|---|
| Dosed detergent/bleach | 4 g/l | 7 g/l |
| sud volume per beaker (ml) | 250 | 200 |
| temperature (°C) | 40 | 30 |
| time (min.) | 30 | 30 |
| detergent | IEC-STPP | IEC-zeolite |
| detergent dosage (g/l) | 3.68 | 5.6 |
| Na-perborate.4aq. (g/l) | 0.32 | 1.4 |
| TAED (mg/l) | 60 | 210 |
| EMPA 116 / 117 (5x5cm) | 2 / 2 | 2 / 2 |
| CFT AS-3 CACAO (5x5cm) | 0 | 2 |
| EMPA 221 clean swatch (10x10cm) | 0 | 2 |
| Stainless steel balls (φ6mm) | 0 | 15 |
| [Ca²⁺ ] (mM) | 2 | 2 |
| [Mg²⁺] (mM) | 0.7 | 0.7 |
| [NaCO₃] (mM) | 2.5 | 0 |

The IEC-STPP detergent powder (IEC Test Detergent Type I, Formulation May 1976) and the IEC-zeolite detergent powder (Formulation April 1988) were purchased from WFK-Testgewebe GmbH, Adlerstraße 44, D-4150, Krefeld, Germany. The performance on cacao was measured on CFT AS-3 swatches (purchased from CFT, Center For Test Materials, PO Box 120, Vlaardingen, The Netherlands). Two mutants, E87S and E87Q, were tested in the IEC-STPP system at 10g/l of STPP/bleach containing powder detergent as indicated in Table II. In addition performance measurements at 4g/l were made in the IEC-STPP system which was slightly modified (indicated as ADE+ in the tables): Instead of 40°C, 30 minutes and 2mM Ca²⁺, the wash performance tests were carried out at 30°C during 20 minutes in the presence of 5mM Ca²⁺. In addition 2 EMPA 221 swatches and 15 stainless steel balls with a 6 mm diameter were included.

The results are summarized in the accompanying Tables I, II, III .

### EXAMPLE 2

In order to determine the wash performance of some of the new PB92 protease mutants under conditions of low detergency to mimic typically U.S. conditions, the wash performance was determined in a washing test similar to the test described in Example 1, but with some modifications. The main characteristics of the test are summarized below:

| | |
|---|---|
| sud volume per beaker (ml) | 200 |
| time (min.) | 20 |
| detergent A dosage (g/l) | 1.3 |
| EMPA 116 / 117 (5x5cm) | 2 / 2 |
| CFT AS-3 cacao (5x5cm) | 2 |
| EMPA 221 clean swatch (10x10cm) | 2 |
| Stainless steel balls (φ6mm) | 15 |
| [Ca²⁺] (mM) | 2 |
| [Mg²⁺] (mM) | 0.7 |

**I. Oxidation resistant PB92 M216 protease mutants - wash performance ≥100%**

| Positions involved: **60, 99, 102, 116, 126, 127, 128, 197, 198, 203, 211** | | | | |
|---|---|---|---|---|
| Protease mutant | STPP 4g/l % | zeolite 7g/l % | k_{cat} 1/s | Kₘ mM |
| PB92 protease (unmodified) | 100 | 100 | 105 | 1.0 |

| PB92 mutant with M2168 and: | | | | |
|---|---|---|---|---|
| N60E | 120 | 76¹¹⁷ | 7 | 2.3 |
| S99G | | 119 | 6 | 1.3 |
| V102A | | 113 | n.d. | n.d. |
| V102L | | 125 | 20 | 2.1 |
| V102N | | 113 | 26 | 4.3 |
| V102P | | 135 | n.d. | n.d. |
| V102S | | 106 | n.d. | n.d. |
| G116V, S126L, P127Q, S128A | 100 | | 23 | 8.7 |
| G116V,S126N,P127S,S128A | 110 | | 7 | 4.4 |
| G116V, S126R, P127Q, S128D | 120 | | 7 | 1.3 |
| P127E,S128T | 160 | 45 | 5 | 1.0 |
| V197T | | 129 | 9 | 1.7 |
| N198G | | 133 | 6 | 1.2 |
| Y203W | | 132 | 13 | 1.8 |

| PB92 mutant with M216Q and: | | | | |
|---|---|---|---|---|
| G116V,S126N,P127S,S128A | 130 | 70 | 3 | 4.5 |
| S126M,P127A,S128G | 100 | | 36 | 5.1 |

| PB92 mutant with M216F and: | | | | |
|---|---|---|---|---|
| V102L | | 135 | 9 | 1.3 |

| | | | | |
|---|---|---|---|---|
| ¹¹⁷ : Performance measured on EMPA 117. n.d.: Not determined | | | | |

**II. Non-oxidation resistant PB92 protease mutants (WP>100%)**

| Positions involved: **87, 97, 99, 102, 116, 117, 126, 127, 128, 130, 133, 134, 154, 156, 158, 160, 164, 166, 169, 175, 180, 182, 193, 197, 198, 203, 211 and 212** | | | | |
|---|---|---|---|---|
| Protease mutant | STPP 4g/l % | zeolite 7g/l % | k_{cat} 1/s | Kₘ mM |
| PB92 protease (unmodified) | 100 | 100 | 105 | 1.0 |
| E87S | 140^{10g/l} | 126 | 134 | 1.7 |
| E87Q | 145^{10g/l} | 115¹¹⁷ | 100 | 1.3 |
| S97D | 160 | | 35 | 0.4 |
| S99G | | 170 | 63 | 0.5 |
| S99G,V102I | | 226 | 202 | 1.2 |
| S99G,V102L | | 209 | 166 | 1.0 |
| S99G,V102N | | 213 | 206 | 2.4 |
| S99G,S130G | | 190 | 64 | 1.2 |
| S99G,Y203W | | 148 | 77 | 0.6 |
| S99T | | 137 | 81 | 1.0 |
| V102A | | 110¹¹⁷ | 23 | 0.3 |
| V102G | | 111 | 217 | 0.5 |
| V102H | | 106 | 78 | 0.6 |
| V102I | | 180 | 252 | 1.3 |
| V102I, G116V, S126V, P127M | | 182 | 206 | 2.5 |
| V102I, S130G | | 180 | 141 | 2.0 |
| V102L | | 180 | 194 | 0.8 |
| V102L, G116V, S126V, P127M | | 147 | 160 | 2.3 |
| V102L, S130G | | 154 | 159 | 1.7 |
| V102M | | 136 | 253 | 1.3 |
| V102N | | 170 | 199 | 2.3 |
| V102N,N198G | | 253 | 223 | 3.0 |
| V102N,N197T,N198G | | 227 | 247 | 3.1 |
| V102N,N198G,Y203W | | 162 | 210 | 2.3 |
| V102N,Y203W | | 178 | 252 | 1.9 |
| V102P | | 145 | 13 | 0.4 |
| V102Q | | 150 | 87 | 1.0 |
| V102S | | 136 | 47 | 0.4 |
| V102T | | 165 | 109 | 0.9 |
| V102Y | | 124 | 275 | 0.3 |
| G116V, S126L, P127Q, S128A, S160D | 200 | | 65 | 9.1 |
| G116V,S126L,P127N,S128V,Y203W | | 138 | 253 | 3.6 |
| G116V,S126N,P127S,S128A | 130 | | 64 | 2.4 |
| G116V,S126V,P127E,S128K,S160D | 175 | | 30 | 4.4 |
| G116V,S126V,P127M,S160D | 235 | | 28 | 3.4 |
| G116V,S126V,P127M,N198G | | 159 | 162 | 1.9 |
| G116V,S126V,P127M,Y203W | | 132 | 186 | 1.4 |
| G116V,S126V,P127M,Y203G | | 108 | 154 | 1.8 |
| S126F,P127A | 130 | | 223 | 10.0 |
| S126F,P127D | 120 | | 112 | 8.2 |
| S126F,P127H | 150 | | 197 | 7.8 |
| S126F,P127N | 200 | | 80 | 3.3 |
| S126F, P127Q | 150 | | 104 | 5.0 |
| S126M,P127A,S128G,S160D | 300 | | 200 | 1.9 |
| S126V,P127M | | 200 | 191 | 1.7 |
| P127E | 200 | 140 | 137 | 1.6 |
| S130G | | 170 | 85 | 1.5 |
| S130G,Y203W | | 142 | 65 | 1.2 |
| L133W | | 125 | 274 | 1.5 |
| L133Y | | 125 | n.d. | 5.9 |
| E134C^{+DTT} | | 170 | n.d. | n.d. |
| S154E | 200^{ADE+} | | 36 | 1.0 |
| S154G | | 110 | 70 | 0.9 |
| S154N | | 133 | 79 | 1.1 |
| A156D | 195^{ADE+} | 120 | 77 | 0.9 |
| A156G | | 104 | 61 | 0.5 |
| S158G | | 105 | 82 | 0.9 |
| S158N | | 138 | 71 | 0.6 |
| S160D,A166D,M169I | 200 | 120 | 13 | 1.2 |
| S160D,N212D | 120 | | 12 | 1.5 |
| S160G | 100 | 115¹¹⁷ | 29 | 1.7 |
| R164M | | 110 | 99 | 1.0 |
| R164V | | 131 | 121 | 1.2 |
| R164Y | | 135 | 115 | 0.8 |
| D175E | | 113 | 99 | 0.9 |
| R180I | | 120 | 106 | 0.9 |
| S182N,Y203T | | 125 | 94 | 0.7 |
| V193A,Y203L | | 132 | 85 | 0.6 |
| V193A,Y203V | | 132 | 86 | 0.6 |
| V197N | | 113 | 99 | 1.0 |
| V197T | | 120 | 146 | 1.1 |
| V197W | | 115 | 62 | 0.9 |
| N198C^{+DTT} | | 124 | n.d. | n.d. |
| N198G | | 152 | 92 | 1.1 |
| N198G,Y203W | | 132¹¹⁷ | 82 | 0.7 |
| N198S | | 125 | 84 | 0.7 |
| N198V | | 121 | 104 | 0.8 |
| Y203E | | 130 | 111 | 0.6 |
| Y203G | | 135 | 91 | 1.1 |
| Y203K | | 108 | 103 | 0.6 |
| Y203L | | 106¹¹⁷ | 132 | 0.6 |
| Y203T | | 135 | 92 | 0.6 |
| Y203V | | 135 | 90 | 0.6 |
| Y203W | | 165 | 144 | 1.0 |
| L211E | | 164 | 9 | 0.9 |
| L211G,N212D | | 105 | 39 | 1.2 |
| L211N,N212D | | 132 | 16 | 0.7 |
| L211V,N212D | | 106 | 26 | 1.4 |
| L211Y,N212S | 123 | | 81 | 0.5 |
| N212E | 140 | | 94 | 1.2 |

| | | | | |
|---|---|---|---|---|
| ¹¹⁷ : Performance measured on EMPA 117. n.d.: Not determined | | | | |

**III. PB92 protease mutants and their performance on cacao**

| Positions involved: **102, 116, 117, 126, 127, 128, 133, 154, 156, 158, 159, 160, 164, 197, 198, 203, 211 and 216** | | | | | |
|---|---|---|---|---|---|
| PB92 protease mutant | Wash Performance zeolite at 7g/l(%) | | | Kinetic parameters | |
| | | | | k_{cat} | Kₘ |
| | 116 | 117 | choc | 1/s | mM |
| V102E | 87 | | 133 | 55 | 2.2 |
| V102N, R164Y | 108 | 87 | 124 | 247 | 2.8 |
| V102N,L211E | 101 | 80 | 142 | 48 | 2.8 |
| G116V,S126L,P127N,S128V,A156E | 108 | 73 | 118 | 170 | 2.5 |
| M117L | 126 | 120 | 147 | 64 | 0.7 |
| P127E,Y203W | 105 | 103 | 134 | 135 | 1.0 |
| P127E,L211E | 63 | 47 | 119 | 9 | 1.1 |
| L133I | 126 | | 135 | 43 | 0.7 |
| L133M | 113 | | 126 | 108 | 0.6 |
| S154D, S160G | 109 | | 116 | 32 | 1.7 |
| S154G, S160G | 124 | | 132 | 34 | 2.2 |
| A156E | 140 | 137 | 173 | 105 | 1.3 |
| S158D | 139 | 126 | 190 | 91 | 1.1 |
| S158E | 123 | 121 | 176 | 101 | 1.1 |
| S158E, I159L | 118 | 132 | 132 | 90 | 1.0 |
| S160E | 104 | 110 | 145 | 17 | 0.5 |
| R164I | 119 | 117 | 126 | 127 | 1.1 |
| V197L | 79 | 106 | 119 | 60 | 0.8 |
| N198D | 110 | 110 | 153 | 92 | 0.8 |
| N198E | 102 | 123 | 159 | 87 | 0.7 |
| N198Q | 100 | 111 | 110 | 64 | 0.7 |
| Y203C^{+DTT} | 95 | 107 | 129 | n.d. | n.d. |
| PB92 mutant with **M2168** and: | | | | | |
| | | | | | |
| V102Q | 96 | 87 | 106 | n.d. | n.d. |
| L211E | 100 | | 127 | 2 | 1.1 |

| | | | | | |
|---|---|---|---|---|---|
| ¹¹⁶ : Performance measured on EMPA 116; ¹¹⁷ : Performance measured on EMPA 117. ^{choc} : Performance measured on CFT AS-3 n.d.: Not determined | | | | | |

The composition of Detergent A was as follows:

| ingredients | % by weight |
|---|---|
| alcohol ethoxylate | 13% |
| LAS-90 | 7% |
| polyacrylate | 1% |
| zeolite | 35% |
| Na-silicate | 3% |
| Na₂CO₃ | 20% |
| tri-Na-citrate.2H₂O | 4% |
| Na₂SO₄ | 8% |
| water | to 100% |

Prior to addition of PB92 protease or mutants thereof, the pH of the wash liquor was adjusted to 10.2. The results are shown in Table IV.

In addition the wash performance of some of the mutants was determined at lower temperature. The results at 20°C are shown in table IV. All the mutants which are shown perform significantly better at 20°C than does the wild type under these conditions. Very surprisingly some of the mutants, such as [V102N, S99G], [V102N], [G116V, S126V, P127M, S160D] do show a better wash performance at 20°C than at 30°C. This is opposite to what was expected from the behaviour of wild type PB92 : The wash performance of PB92 goes down upon lowering the laundering temperature. So it seems that our approach to improve the wash performance of an alkaline protease by site specific engineering can also shift the temperature at which these proteases exhibit optimal performance.

**Table IV : Wash performance new PB92 mutants at different temperatures:**

| wash performance (%) | | |
|---|---|---|
| PB92 protease mutants | temperature | |
| | 30°C | 20°C |
| S99G | 123 | n.d. |
| S99G, S130G | 188 | 173¹¹⁷ |
| V102I, S99G | 117¹¹⁷ | n.d. |
| V102N, S99G | 163 | 181 |
| V102N, N198G | 168 | 169¹¹⁷, 155^{choc} |
| V102N, Y203W | 165 | 131 |
| V102N, V197I, N198G | 139¹¹⁷ | n.d. |
| V102N | 146 | 165¹¹⁷ |
| V102I | 121¹¹⁷ | n.d. |
| V102L | 124¹¹⁷ | n.d. |
| S126V, P127M | 179¹¹⁷ | n.d. |
| S126F, P127N, | 147¹¹⁷ | n.d. |
| S126V, P127M, G116V, S160D | 156 | 185 |
| S126L, P127Q, S128A, G116V, S160D | 212 | 187 |
| S126M, P127A, S128G, S160D | 158 | 143¹¹⁷ |
| P127E | 103, 130^{choc} | n.d. |
| S130G | 132 | n.d. |

| | | |
|---|---|---|
| ¹¹⁷ : performance measured on EMPA 117 ^{choc} : performance measured on CFT AS-3 n.d.: not determined | | |

In all experiments the wash performance was determined relative to the PB92 wild type protease. In addition to the above-mentioned detergent A, the wash performance was also determined in several commercial U.S. detergents. The wash results were similar.

All publications (including patent applications) mentioned in this specification are indicative to the level of skill of those skilled in the art to which this invention pertains. All publications are herein incorporated by reference to the same extent as if each individual publication was specifically and individually indicated to be incorporated by reference.

Although the foregoing invention has been described in some detail by way of illustration and example for purposes of clarity of understanding, it will be apparent to one of ordinary skill in the art that many changes and modifications can be made thereto without departing from the spirit or scope of the appended claims.

## Claims

1. A mutant protease for use in detergents
having greater than 90% homology with either the amino acid sequence of PB92 serine protease having the amino acid sequence: 
H₂N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-P-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
or the amino acid sequence of Subtilisin 309 serine protease having the amino acid sequence : 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-S-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
 in which the amino acid residue at a selected site corresponding to position V102 in said PB92 serine protease or said Subtilisin 309 serine protease is changed to A, E, G, H, I, L, M, N, P, Q, S, T, or Y,
having improved wash performance relative to said PB92 serine protease or said Subtilisin 309 serine protease, said improved wash performance being determined in a washing system having the following features: IEC-zeolite detergent Formulation April 1988 5.6 g/l ; sud volume per beaker 200 ml; temperature 30°C ; time 30 min ; Na-perborate 4aq. 1.4 g/l ; TAED 210 mg/l ; 2 EMPA 221 10 × 10cm clean swatch; 15 Stainless steel balls (phi. 6 mm) ; 2mM Ca²⁺ ; 0.7 mM Mg²⁺; 0 mM NaCO₃ ; and 2 EMPA 116 or 2 EMPA 117 or 2 CFT As-3 CACAO 5 x 5 cm swatches ;

2. A mutant protease according to claim 1, which differs from said PB92 serine protease or said Subtilisin 309 serine protease by at least one of the following mutations: [S99G, V102I], [S99G, V102L] , [S99G,V102N], [V102A], [102A, M216S], [V102E], [V102G], [V102H], [V102I], [V102I, G116V, S126V, P127M], [V102I, G116V, S126V, P127M], [V102I, S130G], [V102L], [V102L, G116V, S126V, P127M], [V102L, S130G] [V102L, M216F], [V102L,M216S], [V102M], [V102N], [V102N,R164Y], [V102N, N198G], [V102N, N197T, N198G], [V102N, N198G, Y203W], [Y102N,Y203W), [V102N, L211E], [V102N,M216X, where X is any amino acid except M], [V102N,M216S], [V102P], [V102P, M216S], [V102Q], [V102Q, M216S], [V102S], [V102S, M216S], [V102T], and [V102Y].

3. A PB9.2 mutant protease according to claim 1, which has a mutation at amino acid 102 and at least one other amino acid position.

4. A PB92 mutant protease according to claim 3, which is selected from the group consisting of [S99G, V102N) and [V102N, N198G].

5. A mutant protease according to any one of claims 1 to 4 which is in substantially pure form.

6. A DNA sequence encoding a mutant protease as defined in any one of claims 1 to 4.

7. A method of preparing a mutant protease as defined in any one of claims 1 to 5, which comprises ;
proving a microorganism host strain transformed with an expression vector comprising a DNA sequence encoding a mutant protease whereby said mutant protease is produced, and
recovering said mutant protease.

8. A detergent additive comprising one or more mutant proteases according to any one of claims 1 to 5 and, if desired, one or more enzymes selected from the group consisting of amylases, cellulases and lipases.

9. A detergent composition comprising one or more mutant proteases according to any one of Claims 1 to 5 and, if desired, one or more enzymes selected from the group consisting of amylases, cellulases and lipases.

10. Use of a mutant protease according to any one of claims 1 to 5, in a washing process at a temperature preferably in the rage of about 15°C to about 45°C.

## Patentansprüche

1. Mutante Protease zur Verwendung in Detergentien mit mehr als 90 % Homologie mit entweder der Aminosäuresequenz von der PB92-Serinprotease mit der folgenden Aminosäuresequenz: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-E-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
 oder der Aminosäuresequenz von Subtilisin-309-Serinprotease mit der folgenden Aminosäuresequenz: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-S-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
 in welcher der Aminosäurerest an einer ausgewählten Stelle, die der Position V102 in der PB92-Serinprotease oder der Subtilisin-309-Serinprotease entspricht, zu A, E, G, H, I, L, M, N, P, Q, S, T oder Y abgeändert wird,
mit verbesserter Waschleistung im Vergleich zu jener der PB92-Serinprotease oder der Subtilisin-309-Serinprotease, wobei die verbesserte Waschleistung in einem Waschsystem mit den folgenden Merkmalen bestimmt wird: IEC-Zeolith-Detergenz-Formulierung April 1988, 5,6 g/l; Seifenschaumvolumen pro Becherglas 200 ml; Temperatur 30°C; Zeit 30 min; Na-Perborat.4äq. 1,4 g/l; TAED 210 mg/l; 2 EMPA 221 10 x 10 cm große Reinigungs-Stoffprobe; 15 Kugeln aus nicht-rostendem Stahl (phi: 6 mm), 2 mM Ca²⁺; 0,7 mM Mg²⁺; 0 mM NaCO₃; und 2 EMPA 116 oder 2 EMPA 117 oder 2 CFT As-3 CACAO 5 x 5 cm große Stoffproben;

2. Mutante Protease gemäß Anspruch 1, welche sich von der PB92-Serinprotease oder der Subtilisin-309-Serinprotease durch mindestens eine der folgenden Mutationen unterscheidet:
[S99G, V102I], [S99G, V102L], [S99G, V102N], [V102A], [V102A, M216S], [V102E], [V102G], [V102H], [V102I], [V102I, G116V, S126V, P127M], [V102I, G116V, S126V, P127M], [V102I, S130G], [V102L], [V102L, G116V, S126V, P127M], [V102L, S130G], [V102L, M216F], [V102L, M216S], [V102M]; [V102N], [V102N, R164Y], [V102N, N198G], [V102N, N197T, N198G], [V102N, N198G, Y203W], [V102N, Y203W], [V102N, L211E], [V102N, M216X, worin X eine beliebige Aminosäure außer M ist], [V102N, M216S], [V102P], [V102P, M216S], [V102Q], [V102Q, M216S], [V102S], [V102S, M216S], [V102T] und [V102Y].

3. Mutante PB92-Protease gemäß Anspruch 1, welche eine Mutation bei der Aminosäure 102 und mindestens einer anderen Aminosäureposition besitzt.

4. Mutante PB92-Protease gemäß Anspruch 3, welche aus der Gruppe gewählt wird, die aus [S99G, V102N] und [V102N, N198G] besteht.

5. Mutante Protease gemäß mindestens einem der Ansprüche 1 bis 4, welche im wesentlichen in reiner Form ist.

6. DNA-Sequenz, die eine mutante Protease codiert, wie sie in mindestens einem der Ansprüche 1 bis 4 definiert ist.

7. Verfahren zur Herstellung einer mutanten Protease, wie sie in mindestens einem der Ansprüche 1 bis 5 definiert ist, welches folgendes umfasst:
das Wachsen lassen eines Mikroorganismus-Wirtsstammes, der mit einem Expressionsvektor transformiert ist, umfassend eine DNA-Sequenz, die eine mutante Protease codiert, wobei die mutante Protease hergestellt wird, und
das Gewinnen der mutanten Protease.

8. Detergenzadditiv, umfassend eine oder mehrere mutante Proteasen gemäß mindestens einem der Ansprüche 1 bis 5 und, sofern erwünscht, ein oder mehrere Enzyme, die aus der Gruppe gewählt ist bzw. sind, welche aus Amylasen, Cellulasen und Lipasen besteht.

9. Detergenzzusammensetzung, umfassend eine oder mehrere mutante Proteasen gemäß mindestens einem der Ansprüche 1 bis 5 und, sofern erwünscht, ein oder mehrere Enzyme, die aus der Gruppe gewählt ist bzw. sind, welche aus Amylasen, Cellulasen und Lipasen besteht.

10. Verwendung einer mutanten Protease gemäß mindestens einem der Ansprüche 1 bis 5, in einem Waschverfahren bei einer Temperatur vorzugsweise im Bereich von etwa 15°C bis etwas 45°C.

## Revendications

1. Protéase mutante destinée à être utilisée dans des détergents, ayant une homologie supérieure à 90% avec soit la séquence d'acides aminés de la sérine-protéase PB92 ayant la séquence d'acides aminés : 
H2N-A-Q-S-V-P-W-G-I-S-R-V-Q-A-P-A-A-H-N-R-G-L-T-G-S-G-V-
K-V-A-
V-L-D-T-G-I-S-T-H-P-D-L-N-I-R-G-G-A-S-F-V-P-G-E-P-S-T-
Q-D-G-N-
G-H-G-T-H-V-A-G-T-I-A-A-L-N-N-S-I-G-V-L-G-V-A-P-N-A-E-
L-Y-A-V-
K-V-L-G-A-S-G-S-G-S-V-S-S-I-A-Q-G-L-E-W-A-G-N-N-G-M-H-
V-A-N-L-
S-L-G-S-P-S-P-S-A-T-L-E-Q-A-V-N-S-A-T-S-R-G-V-L-V-V-A-
A-S-G-N-
S-G-A-G-S-I-S-Y-P-A-R-Y-A-N-A-M   A-V-G-A-T-D-Q-N-N-N-R-
A-S-F-S-
Q-Y-G-A-G-L-D-I-V-A-P-G-V-N-V-Q-S-T-Y-P-G-S-T-Y-A-S-L-
N-G-T-S-
M-A-T-P-H-V-A-G-A-A-A-L-V-K-Q-K-N-P-S-W-S-N-V-Q-I-R-N-
H-L-K-N-
T-A-T-S-L-G-S-T-N-L-Y-G-S-G-L-V-N-A-E-A-A-T-R-COOH;
 soit la séquence d'acides aminés de la sérine-protéase Subtilisine 309 ayant la séquence d'acides aminés : 
H2N-A-Q-S-V-P-W-G-I-S-R-V-Q-A-P-A-A-H-N-R-G-L-T-G-S-G-V-
K-V-A-
V-L-D-T-G-I-S-T-H-P-D-L-N-I-R-G-G-A-S-F-V-P-G-E-P-S-T-
Q-D-G-N-
 
G-H-G-T-H-V-A-G-T-I-A-A-L-N-N-S-I-G-V-L-G-V-A-P-S-A-E-
L-Y-A-V-
K-V-L-G-A-S-G-S-G-S-V-S-S-I-A-Q-G-L-E-W-A-G-N-N-G-M-H-
V-A-N-L-
S-L-G-S-P-S-P-S-A-T-L-E-Q-A-V-N-S-A-T-S-R-G-V-L-V-V-A-
A-S-G-N-
S-G-A-G-S-I-S-Y-P-A-R-Y-A-N-A-M   A-V-G-A-T-D-Q-N-N-R-
A-S-F-S-
Q-Y-G-A-G-L-D-I-V-A-P-G-V-N-V-Q-S-T-Y-P-G-S-T-Y-A-S-L-
N-G-T-S-
M-A-T-P-H-V-A-G-A-A-A-L-V-K-Q-K-N-P-S-W-S-N-V-Q-I-R-N-
H-L-K-N-
T-A-T-S-L-G-S-T-N-L-Y-G-S-G-L-V-N-A-E-A-A-T-R-COOH;
 dans laquelle le résidu d'acides aminés à un site sélectionné correspondant à la position V102 dans ladite sérine-protéase PB92 ou ladite sérine-protéase Subtilisine 309 est déplacé en A, E, G, H, I, L, M, N, P, Q, S, T, ou Y;
ayant une performance au lavage améliorée par rapport à ladite sérine-protéase PB92 ou ladite sérine-protéase Subtilisine 309, ladite performance au lavage améliorée étant déterminée dans un système de lavage ayant les caractéristiques suivantes : détergent IEC-zéolite Formulation, avril 1988 à 5,6 g/l; volume de lessive de savon par bécher 200 ml; température 30°C; temps 30 min ; perborate de sodium.4.aq 1,4 g/l ; TAED 210 mg/l; 2 échantillons propres EMPA 221 de 10 x 10 cm; 15 billes en acier inoxydable (phi. 6 mm) ; 2 mM de Ca²⁺ ; 0,7 mM de Mg²⁺ ; 0 mM de NaCO₃ ; et des échantillons 2 EMPA 116 ou 2 EMPA 117 ou 2 CFT As-3 CACAO de 5 x 5 cm.

2. Protéase mutante selon la revendication 1, qui diffère de ladite sérine-protéase PB92 ou de ladite sérine-protéase Subtilisine 309 par au moins une des mutations suivantes :
[S99G, V102I] , [S99G, V102L], [S99G, V102N] , [V102A] , [V102A, M216S], [V102E] , [V102G] , [V102H], [V102I], [V102I, G116V, S126V, P127M], [V102I, G116V, S126V, P127M], [V102I, S130G], [V102L], [V102L, G116V, S126V, P127M] , [V102L, S130G] , [V102L, M216F] , [V102L, M216S] , [V102M] , [V102N] , [V102N, R164Y], [V102N, N198G], [V102N, N197T, N198G], [V102N, N198G, Y203W], [V102N, Y203W], [V102N, L211E], [V102N, M216X, où X est tout acide aminé excepté M], [V102N, M216S], [V102P], [V102P, M216S], [V102Q], [V102Q, M216S], [V102S], [V102S, M216S], [V102T] et [V102Y].

3. Protéase mutante PB92 selon la revendication 1, qui a une mutation à l'acide aminé 102 et à au moins une autre position d'acide aminé.

4. Protéase mutante PB92 selon la revendication 3, qui est sélectionnée dans le groupe composé de [S99G, V102N] et [V102N, N198G].

5. Protéase mutante selon l'une quelconque des revendications 1 à 4 qui est sous une forme sensiblement pure.

6. Séquence d'ADN codant pour une protéase mutante tel que définie dans l'une quelconque des revendications 1 à 4.

7. Procédé de préparation d'une protéase mutante tel que définie dans l'une quelconque des revendications 1 à 5, qui comprend :
la croissance d'une souche hôte de micro-organisme transformée avec un vecteur d'expression comprenant une séquence d'ADN codant pour une protéase mutante dans laquelle ladite protéase mutante est produite, et
la récupération de ladite protéase mutante.

8. Additif détergent comprenant une ou plusieurs protéases mutantes selon l'une quelconque des revendications 1 à 5 et, éventuellement, une ou plusieurs enzymes sélectionnée(s) dans le groupe consistant en amylases, cellulases et lipases.

9. Composition détergente comprenant une ou plusieurs protéases mutantes selon l'une quelconque des revendications 1 à 5 et, éventuellement, une ou plusieurs enzymes sélectionnée(s) dans le groupe consistant en amylases, cellulases et lipases.

10. Utilisation d'une protéase mutante selon l'une quelconque des revendications 1 à 5, dans un procédé de lavage à une température située de préférence dans la plage comprise entre environ 15°C et environ 45°C.
